# DEMANDE DE BREVET EUROPEEN

(11) **EP 3 195 816 A1**
(43) Date de publication de la demande: **26.07.2017**
(21) Numéro de dépôt: 17152352.5
(22) Date de dépôt: 20.01.2017
(51) Int. Cl.: A61B 17/15

(54) **KIT D'OSTÉOTOMIE ET DE POSITIONNEMENT DE FRAGMENTS OSSEUX POUR RECONSTRUCTION AVEC OSTÉOSYNTHÈSE**

(30) Priorité: 20.01.2016 BE 201605044
(71) Demandeur: ADDIPARTS SPRL, 7000 Mons (BE)
(72) Inventeur: Sasserath, Christophe, 7000 Mons (BE); Adam, Nicolas, 59300 Famars (FR); Demoulin, Charles, 7000 Mons (BE); Casimiro, Roméo, 69230 Saint Genis Laval (FR)
(74) Mandataire: Pronovem

(57) **Abrégé**

La présente invention concerne un kit d'ostéotomie et de positionnement et d'ostéosynthèse de fragments osseux comprenant :
- un premier guide de coupe (1,100) comprenant des moyens de fixations (2) à un os (20) à découper en vue d'une ostéosynthèse ledit guide de coupe comprenant au moins deux fentes (3) définissant au moins deux plans de coupe;
- au moins un support (10,110) de fragment osseux (30), ledit support (10, 110) de fragment osseux comprenant des moyens de fixation (16) au fragment osseux ;
- des moyens de positionnement univoque (14, 15,114) du au moins un support (10,110) de fragment osseux (30) par rapport à un os à traiter,
ledit guide de coupe (1, 110) comprenant des moyens de positionnement univoque(4,5,104) coopérant avec des moyens de positionnement univoque (14, 15,114) correspondants sur le au moins un support (10, 110) de fragment osseux (30).

## Description

### Objet de l'invention

La présente invention se rapporte à un ensemble sur mesure d'outils de guidage de coupe et de positionnement de fragments osseux facilitant l'ostéosynthèse.

### Etat de la technique

De nombreux actes chirurgicaux nécessitent une intervention de reconstruction osseuse, que ce soit en chirurgie humaine ou animale. Ces interventions comprennent une première étape d'ostéotomie, dans laquelle un fragment d'os à traiter est retiré et/ou libéré en vue d'une mobilisation ; une seconde étape dans laquelle soit un fragment d'os sain est prélevé ailleurs dans le squelette du patient, soit un fragment osseux préalablement libéré est mobilisé et déplacé ; et enfin une étape d'ostéosynthèse dans laquelle au moins un fragment d'os sain est positionné et fixé pour reconstruire l'os à traiter.

Parmi les interventions de reconstruction osseuse, notons la reconstruction maxillaire et mandibulaire, la reconstruction cranio-faciale, la cranioplastie pour la reconstruction de voute crânienne, la reconstruction orbitomalaire ou centrofaciale, la chirurgie orthognatique, et divers interventions en orthopédie, telles que l'ostéotomie tibiale de valgisation, l'ostéotomie fémorale, allongeant, varisation, valgisation ou encore la reconstruction des membres après résection tumorale.

Aujourd'hui, ces interventions sont généralement planifiées à partir de représentations 3D à la fois de l'os à traiter et de l'os utilisé pour l'ostéosynthèse (greffons). Ces représentations 3D peuvent, par exemple, être obtenues à partir de données d'un scanner ou d'une IRM. Cette planification est généralement accompagnée d'une simulation des différentes étapes de l'intervention, en particulier, la position et les angles des différents plans de coupe, ainsi que le repositionnement des fragments osseux.

Afin d'aider le chirurgien à respecter au plus près le plan chirurgical préétabli, en particulier les plans de coupe, des guides de coupe personnalisés sur mesure ont été développés. Ces guides de coupe présentent des surfaces de fixation aux os épousant la forme de ceux-ci, ce qui permet de positionner les plans de coupe de façon précise et univoque.

Néanmoins, ces méthodes de l'art antérieur ne se préoccupent généralement que de la précision des découpes, laissant la reconstruction à l'appréciation du chirurgien, ce qui pose des problèmes de précision lors de la reconstruction. Il est également alors nécessaire de manipuler les fragments directement pour les adapter dans la reconstruction ce qui implique des risques de dégâts osseux et vasculaires et augmente le temps opératoire de manière significative.

Ce problème est particulièrement aigu lorsque la reconstruction est faite à partir de plusieurs fragments pouvant malencontreusement être intervertis, inversés, tournés, ...

Actuellement, il existe une solution consistant à réaliser une plaque de reconstruction en titane sur mesure dont le repositionnement doit se faire en replaçant les fragments dans la plaque et en vissant les fragments dans des trous préalablement réalisés. Cela reste des manipulations difficiles car il n'est pas toujours aisé de retrouver les trous de forage dans les greffons.

### Buts de l'invention

La présente invention vise à améliorer la précision du positionnement des fragments osseux lors d'une reconstruction ou d'un déplacement osseux selon un planning prédéfini et à faciliter l'ostéosynthèse des fragments en améliorant leur positionnement conformément à une planification prédéterminée.

La présente invention vise également à réduire les manipulations de fragments osseux lors du positionnement et/ou d'une ostéosynthèse.

La présente invention permet également de stabiliser les fragments osseux lors de la fixation d'une plaque d'ostéosynthèse.

D'autres avantages de l'invention apparaîtront également dans la description détaillée de l'invention.

### Résumé de l'invention

La présente invention concerne un kit d'ostéotomie et de positionnement de fragments osseux comprenant :
a. un premier guide de coupe comprenant des moyens de fixations à un os à découper en vue d'une ostéosynthèse ledit guide de coupe comprenant au moins deux fentes définissant au moins deux plans de coupe;
b. au moins un support de fragment osseux, ledit support de fragment osseux comprenant des moyens de fixation au fragment osseux ;
c. des moyens de positionnement univoque du au moins un support de fragment osseux par rapport à un os à traiter, ledit guide de coupe comprenant des moyens de positionnement univoque coopérant avec des moyens de positionnement univoque correspondants sur le au moins un support de fragment osseux.

Selon des modes préférés de l'invention, le kit d'ostéotomie et de positionnement de fragments osseux de l'invention comporte au moins une, ou une combinaison appropriée des caractéristiques suivantes :
- le premier guide de coupe comprend des moyens de fixation amovibles du au moins un support de fragment osseux ;
- les moyens de positionnement univoque du au moins un support de fragment osseux comprennent un guide de reconstruction comprenant :
   ∘ des moyens de positionnement univoque coopérant avec les moyens de positionnement univoque correspondant sur le au moins un support de fragment osseux et des moyens de fixation amovible du au moins un support de fragment osseux;
   ∘ et des moyens de fixation dans une position prédéfinie à un os à traiter;
- les moyens de fixation dans une position prédéfinie à un os à traiter comprennent des éléments d'ancrage destinés à être fixés à une position prédéfinie sur un os à traiter et permettant une fixation amovible dans une position prédéterminée du guide d'ostéosynthèse aux éléments d'ancrage ;
- le kit d'ostéotomie et de positionnement de fragments osseux comprend un second guide de coupe pouvant être fixé de façon amovible dans une position prédéterminée sur les éléments d'ancrage ;
- les moyens de fixation amovible sont sélectionnés parmi le groupe consistant en moyens d'encliquetage, moyens d'enclipsage, moyens de fixation par pression et système tenon/mortaise ;
- le kit d'ostéotomie et de positionnement de fragments osseux comprend une pluralité de supports de fragments osseux, les moyens de positionnement univoque de chacun de ces supports étant différents les uns des autres.

Un second aspect de l'invention concerne une méthode de fabrication d'un kit d'ostéotomie et de positionnement de fragments osseux selon l'invention comprenant les étapes de :
a) obtenir une représentation 3D d'un os à reconstruire;
b) obtenir une représentation 3D d'un os pour le prélèvement de greffons de reconstruction en vue d'ostéosynthèse (os pour ostéosynthèse);
c) définir un plan chirurgical comprenant des plans de coupe de l'os à reconstruire et de l'os pour ostéosynthèse, lesdits plans de coupe définissant des fragments osseux, et le plan chirurgical comprenant le positionnement relatifs de l'os à reconstruire d'une part et des fragments d'osseux d'autre part;
d) déterminer la forme 3D d'un premier guide de coupe comprenant une surface de positionnement sur l'os d'ostéosynthèse et des fentes correspondantes aux plans de coupe définis à l'étape (c);
e) déterminer la forme 3D d'au moins un support de fragment osseux, positionné de façon univoque sur le premier guide de coupe, et ayant donc une position prédéterminée par rapport aux fragments osseux, la forme 3D du support de fragment comprenant des moyens de positionnement univoque du au moins un fragment osseux par rapport à l'os à reconstruire;
f) produire les formes 3D du premier guide de coupe et des supports de fragments osseux.

Avantageusement, les formes 3D sont produites par un procédé d'impression 3D.

### Brève description des figures

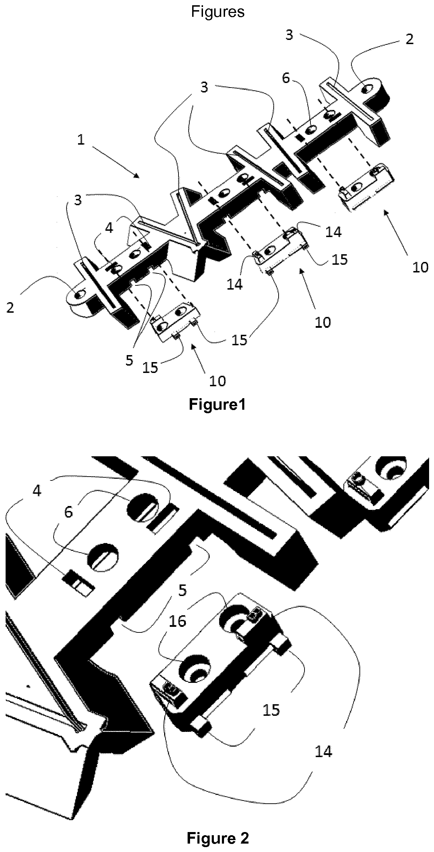

La figure 1 représente un exemple de réalisation d'un guide de coupe avec des éléments référentiels et des supports de fragments osseux selon l'invention.

La figure 2 représente un agrandissement du système référentiel utilisé dans le guide de coupe et des supports de fragments osseux de la figure 1.

La figure 3 représente un exemple de réalisation d'un guide de reconstruction osseux selon l'invention.

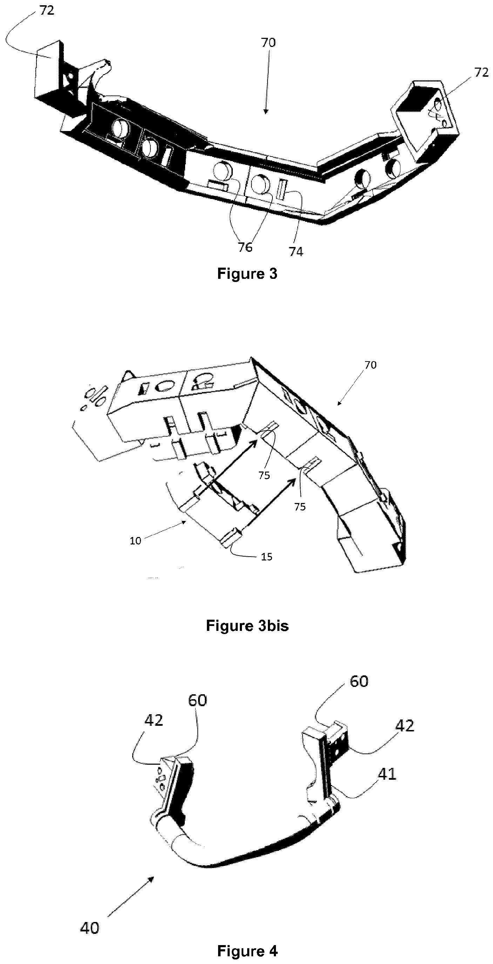

La figure 3 bis représente un exemple de réalisation d'un guide de reconstruction osseux et son assemblage avec un support de greffons osseux.

La figure 4 représente un exemple de réalisation d'un guide de coupe pour la découpe de l'os à traiter.

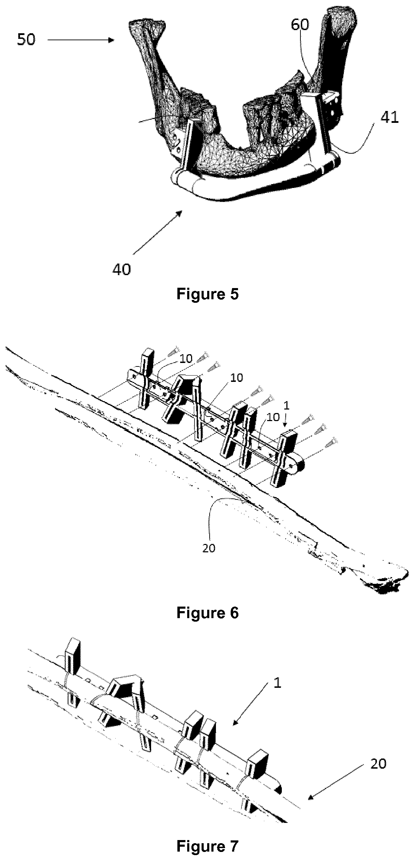

La figure 5 représente un guide de coupe selon l'invention placé sur un os à traiter.

La figure 6 représente la pose d'un exemple de guide de coupe du greffon osseux et des supports de fragments osseux sur un greffon ou sur l'os à déplacer.

La figure 7 représente un exemple de guide de coupe fixé sur l'os à reconstruire permettant la résection de l'os à ôter ou à déplacer.

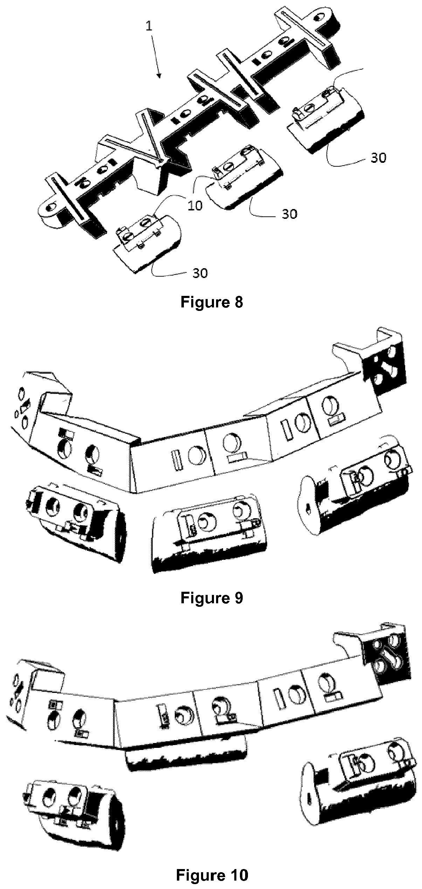

La figure 8 représente un exemple de guide de coupe et des supports référentiels de fragments osseux après démontage du guide de coupe, et séparation des fragments osseux.

Les figures 9 à 11 représentent les étapes d'assemblage des fragments osseux sur le guide de reconstruction en vue de l'ostéosynthèse.

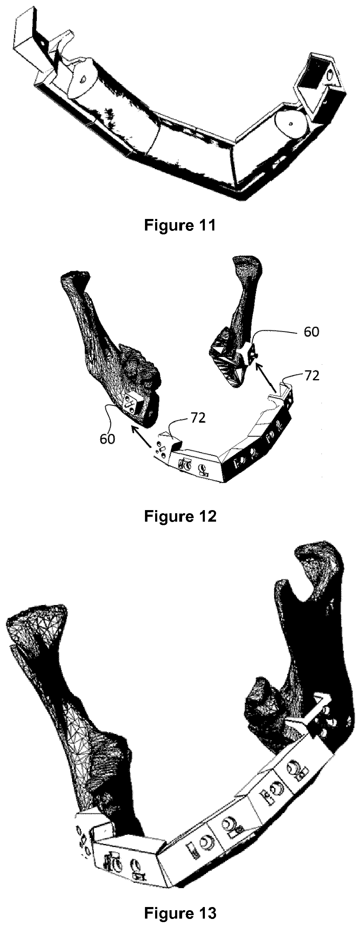

La figure 12 représente l'étape de fixation du guide de reconstruction en vue de l'ostéosynthèse sur l'os à traiter.

La figure 13 représente le guide de reconstruction en vue de l'ostéosynthèse fixé sur l'os à traiter.

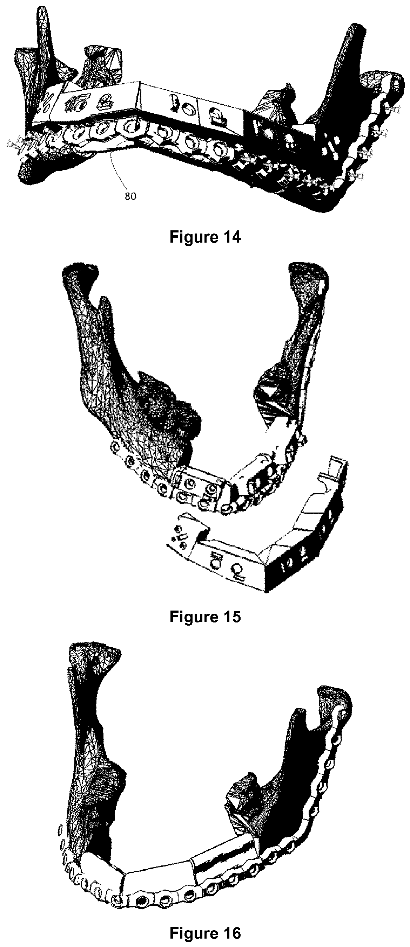

La figure 14 représente l'étape de fixation d'une plaque d'ostéosynthèse fixant de façon permanente les fragments osseux à l'os à traiter.

La figure 15 représente l'étape de retrait du guide de reconstruction en vue de l'ostéosynthèse et des supports de fragments d'os.

La figure 16 représente l'os reconstruit en fin d'intervention.

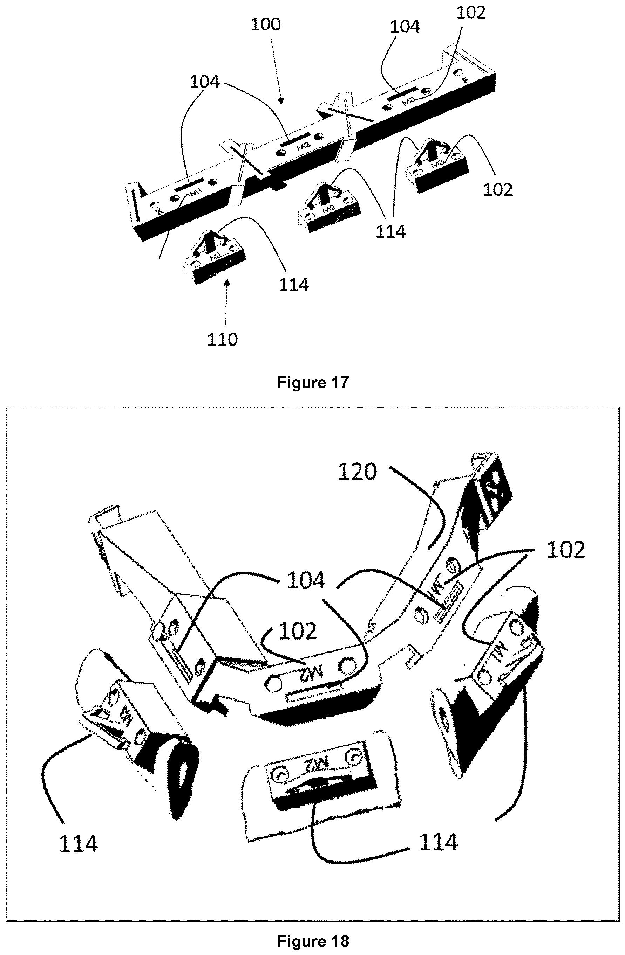

La figure 17 représente un autre exemple de géométrie de moyens de positionnement des supports de fragments osseux.

La figure 18 représente un autre exemple de géométrie de moyens de positionnement des supports de fragments osseux sur un guide de reconstruction.

### Références numériques des figures

1. Guide de coupe de greffon osseux ou os à mobiliser
2. Trou de fixation du guide de coupe de greffon osseux ou os à mobiliser
3. Fente de guidage de coupe
4. Lumière de positionnement des éléments de support de fragment d'os (mortaise, détrompeur)
5. Lumière latérale pour le positionnement et le détrompage de support de fragment d'os
6. Trou permettant le passage de vis de fixation des supports de fragments d'os
10. Support de fragments de greffon osseux ou os à mobiliser
14. tenon ou détrompeur s'engageant dans la lumière 4 correspondante
15. tenon ou détrompeur latéral s'engageant dans la lumière 5 correspondante
16. trou de vis de fixation des supports de fragments de greffon osseux
20. Os pour ostéosynthèse ou os à mobiliser
30. fragments de greffon osseux ou d'os à mobiliser
40. Guide de coupe d'ostéotomie pour la découpe de l'os à traiter
41. Fente guide de coupe
42. Moyens de fixation à des moyens d'ancrages 60
50. Os à traiter
60. Moyens d'ancrage à l'os à traiter 50
70. guide de reconstruction en vue de l'ostéosynthèse
72. moyens de fixation et de positionnement aux moyens d'ancrage 60
74, 75. Lumière de positionnement des supports de fragments osseux 10, 110
76. Trou permettant le passage de vis de fixation des supports 10, 110 de fragments d'os
80. Plaque d'ostéosynthèse
100. Guide de coupe d'ostéotomie pour la découpe de l'os à traiter
102. repères visuels de numérotation
104. lumière
110. support
114. clip mâle
120. guide de reconstruction en vue de l'ostéosynthèse

### Description détaillée de l'invention

La présente invention concerne un kit d'outillage pour la découpe et le positionnement précis de fragments osseux pour des interventions comprenant une étape d'ostéosynthèse. Ce type d'intervention comprend généralement trois étapes :
i. découpe d'un os à traiter selon des plans de coupe prédéfinis;
ii. prélèvement de fragment(s) osseux sur un os pour ostéosynthèse (par exemple le péroné) selon des plans de coupe prédéfinis;
iii. placement du (des) fragment(s) osseux (greffons) entre les plans de coupe définis à l'étape i.

Dans la présente invention, des supports 10,110 de fragments osseux (greffons) 30 sont placés à une position relative prédéfinie par rapport aux plans de coupe, en les fixant de façon amovible, avant la découpe à un guide de coupe 1,100. Ces supports, fixés aux greffons avant la découpe de ceux-ci, serviront ultérieurement de repères de positionnement des fragments osseux lors de la reconstruction à l'aide du guide de reconstruction 70,120.

Par exemple, les supports 10,110 sont clipsés dans une cavité du guide de coupe 1, 100 avant l'intervention. Des repères disposés sur les flancs 15 et/ou sur la surface externe 14 permettent avantageusement de repérer chaque support 10,110 et de les positionner de façon précise sur le guide de coupe. Par surface externe, nous entendons ici la surface opposée à la surface en contact avec l'os 20 lors de l'utilisation. Des cavités ou lumières (4,5) correspondantes sur le guide de coupe permettent un emboitement des supports à une position précise par rapport au guide de coupe 1, 100. Ces repères sont différents pour chaque support et empêchent les permutations/inversions entre les différents supports 10, 110. De cette façon, les supports 10, 110 de fragments osseux 30 sont positionnés de façon précise par rapport aux plans de coupe.

De façon alternative ou complémentaire, le positionnement univoque peut être obtenu par des repères visuels 102 (numérotation, ...) placés à la fois sur le guide de coupe (cf. Fig. 17) et sur le guide de reconstruction (cf. Fig 18).

Avantageusement, les repères 14,15 servent de tenons s'insérant par forçage dans les lumières 4,5 faisant office de mortaises, permettant une fixation de type tenon/mortaise.

De façon alternative (ou complémentaire) tel que représenté à la figure 17 et figure 18, les moyens de fixations des supports de fragments osseux peuvent comprendre un clip mâle 114 venant s'insérer dans une lumière correspondante 104 sur le guide de coupe de l'os (Fig. 17) et le guide de reconstruction (Fig. 18) pour osthéosynthèse.

Des moyens de fixation permettent de fixer les supports 10, 110 sur l'os d'ostéosynthèse avant la découpe. Par exemple, un orifice 16 permet de visser le support sur l'os. Dans ce cas, un trou correspondant 6 d'un diamètre supérieur à la tête de la vis est ménagé dans le guide de coupe pour permettre le vissage lorsque le guide de coupe 1, 100 est en place.

Le guide de coupe 1, 100 et le(s) support 10,110 de fragment(s) osseux 30 présentent une surface interne épousant de préférence la forme tridimensionnelle de l'os pour ostéosynthèse 20, de façon à permettre un positionnement précis et univoque.

Le guide de coupe comprend aussi des moyens de fixation à l'os d'ostéosynthèse. Ces moyens de fixation sont par exemple des trous de vis 2 permettant le vissage du guide de coupe 1, 100 à l'os 20. Là aussi, les surfaces d'ancrages du guide de coupe 1, 100 épousent la surface de l'os 20, toujours dans un souci de positionnement précis.

Les supports 10,110 de fragments d'os 30 comprennent des moyens de positionnement les uns par rapport aux autres et par rapport à l'os à reconstruire 50.

Par exemple, ces supports 10,110 comprennent des tenons et mortaises permettant de les fixer dans une position prédéfinie aux supports adjacents, les fragments extrêmes (i.e. dont au moins une extrémité du fragment d'os se raccorde à un plan de coupe de l'os à traiter) comprenant des moyens de positionnement par rapport à l'os à traiter 50.

De préférence le(s) support(s) 10,110 de fragment(s) osseux est positionné dans un guide de reconstruction 70, 120, comprenant avantageusement des moyens de positionnement 74,75 correspondant aux moyens de positionnement 4,5 du guide de coupe d'ostéosynthèse 1 par rapport aux supports 10,110. Le guide de reconstruction 70, 120 comprend des moyens de fixation et de positionnement 72 sur l'os à traiter. La forme de ce guide permet bien entendu de positionner les fragments osseux 30 dans la position définie par le plan chirurgical.

De préférence, le kit d'outillage de l'invention comprend aussi des moyens d'ancrage 60 fixés sur l'os à traiter 50 en début d'intervention (avant la première étape d'ostéotomie). Ces moyens d'ancrage 60 coopèrent avec les moyens de fixation et de positionnement 72 du guide de reconstruction 70, 120, de façon à fixer et positionner le guide de reconstruction 70, 120 sur l'os à traiter 50.

Avantageusement, un second guide de coupe 40 servant à l'étape d'ostéotomie de l'os à traiter est inclus dans le kit de l'invention. De préférence, ce guide de coupe utilise les mêmes moyens de fixation et de positionnement des fragments osseux que le guide d'ostéosynthèse (ou que les supports 10,110 de fragments d'os 30 si ceux-ci sont directement fixés sur l'os à traiter). Cette identité de moyens de positionnement permet encore d'améliorer la qualité du positionnement du guide de reconstruction 70, 120 par rapport aux plans de coupe de l'os à traiter.

Avantageusement donc, le second guide de coupe 40 comprend des moyens de fixation et de positionnement 42 aux moyens d'ancrages 60. Ces moyens d'ancrage 60 servant aussi au positionnement du guide d'ostéosynthèse 70, 120, ce qui assure un bon alignement entre l'ostéotomie initiale et l'ostéosynthèse ultérieure selon la planification.

Un avantage de l'invention est de pouvoir repositionner les fragments osseux 30 de manière précise et facile selon la planification.

La présente invention permet d'éviter une manipulation excessive des fragments, car par ce système d'assemblage, les fragments 30 se repositionnent sans le moindre risque d'erreur et ne nécessitent plus de manipulations particulières car on ne manipule que le système d'assemblage. Une fois assemblé, il ne reste plus qu'à stabiliser les fragments osseux 30 par des plaques d'ostéosynthèse 80.

La présente invention prévoit la possibilité soit d'adapter des plaques 80 sur site ce qui est aisé car le guide d'ostéosynthèse 70, 120 et les fragments d'os 30 qui y sont fixés par les supports 10,110 sont parfaitement stables, soit par une plaque préformée préalablement et en relation étroite avec le guide 70, 120.

Un autre aspect de l'invention concerne la conception et la fabrication du kit d'outillage de l'invention.

La méthode de fabrication d'un kit d'ostéotomie et de positionnement de fragments osseux selon l'invention comprend une première étape consistant en l'obtention d'une représentation 3D d'un os à reconstruire 50 et d'un os pour ostéosynthèse 20. Ce type de représentation pourra par exemple être extrait des données obtenues par scanner ou par IRM.

Dans un second temps, un plan chirurgical est défini, ce plan chirurgical comprenant les plans de coupe de l'os à reconstruire 50 et de l'os pour ostéosynthèse 20. Les plans de coupe de l'os pour ostéosynthèse 20 définissant les fragments osseux 30. Le plan chirurgical comprend aussi les positionnements relatifs de l'os à reconstruire 50 d'une part et des fragments d'ostéosynthèse 30 d'autre part. Typiquement, ce type de plan chirurgical est obtenu au moyen de logiciel de conception assistée par ordinateur.

Ensuite, avantageusement toujours dans l'environnement ayant servi à produire le plan chirurgical, on détermine la forme 3D d'un premier guide de coupe 1, 100 comprenant une surface de positionnement sur l'os pour ostéosynthèse 20 et des fentes correspondantes aux plans de coupe définis dans le plan chirurgical.

La forme 3D d'au moins un support 10,110 de fragment osseux 30, positionné de façon univoque sur le premier guide de coupe 1, 100 est aussi déterminé à ce stade.

A ce stade, les fragments 30 attachés aux supports 10,110 sont virtuellement repositionnés sur l'os à traiter 50 selon la planification dans l'environnement de conception 3D. Le guide d'ostéosynthèse 70, 120, est alors construit autour des supports 10, 110 dans l'environnement virtuel, de façon à permettre un positionnement univoque des fragments lors de l'intervention réelle. Lors de cette étape, des moyens de fixation 72 et de positionnement univoque du guide de coupe vis-à-vis de l'os à traiter 50 sont définis dans l'environnement virtuel.

De préférence, la méthode de fabrication d'un kit selon l'invention comprend une première étape de conception d'un guide de coupe 40 de l'os à traiter, ce guide de coupe 40 de l'os à traiter comprenant des moyens de fixations 42 analogues aux moyens de fixations 72 présents sur le guide reconstruction 70, 120.

De préférence, les moyens de fixation 42, 72 à la fois du guide de coupe 40, de l'os à traiter et du guide pour ostéosynthèse sont adaptés pour être fixés et positionnés de façon amovible à des moyens d'ancrage 60.

Les moyens d'ancrage 60 sont par exemple prévus pour être vissés à l'os 50 et fixés aux moyens de fixations 42,72 du guide de coupe 40 et du guide de reconstruction 70, 120 par pression ou clipsage, encliquetage, tenon/mortaise, ou tout autre moyen permettant un montage/démontage rapide, précis et aisé.

Enfin, les données tridimensionnelles générées sont utilisées pour produire les formes 3D du premier guide de coupe et des supports de fragments osseux. Cette fabrication est avantageusement effectuée par impression tridimensionnelle ou fabrication additive.

### Exemple

Les figures 5 à 16 représentent les différentes étapes d'utilisation d'un kit selon l'invention dans le cas d'une intervention mandibulaire.

Les premières étapes, non représentées de cet exemple consistent à obtenir une représentation 3D de la mandibule à reconstruire, et à planifier l'intervention.

La figure 5 représente la mise en place du guide de coupe 40 sur la mandibule 50. Dans cette étape, les moyens d'ancrage 60, initialement positionnés dans le logement 42 du guide de coupe, sont vissés sur la mandibule 50. La mandibule est alors découpée selon les plans de coupes définis par les fentes guides 41. Le guide de coupe peut alors être déposé, seuls restant en place les moyens d'ancrages 60, qui serviront ultérieurement à positionner et fixer le guide d'ostéosynthèse 70, 120.

La figure 6 représente l'étape de fixation, au moyen de vis, des supports 10, 110 de fragments d'os et du guide de coupe 1, 100 à l'os pour ostéosynthèse 20. La figure 7 représente le guide après sa mise en place.

La figure 8 représente les fragments d'os 30 sur leurs supports 10, 110 après découpe. Les fragments d'os 30 sont alors ordonnés et positionnés sur le guide d'ostéosynthèse 70, 120 au moyen de leurs supports 10, 110. Ces étapes sont représentées aux figures 9 à 11.

La figure 12 représente la mise en place du guide d'ostéosynthèse 70, 120 sur les moyens d'ancrage 60. Tandis que la figure 13 montre la mandibule avec le guide 70, 120 mis en place.

Après la mise en place du guide, les fragments osseux sont fixés à la mandibule au moyen d'une plaque d'ostéosynthèse 80, vissée aux fragments et à la mandibule, tel que représenté à la figure 14. La plaque peut éventuellement être mise en place sur les fragments avant le repositionnement sur la mandibule.

Enfin, la figure 15 montre le retrait du guide d'ostéosynthèse 70, 120 et des supports de fragments d'os 10, 110 tandis que la figure 16 montre l'os après reconstruction en fin d'intervention.

## Revendications

1. Kit d'ostéotomie et de positionnement de fragments osseux comprenant:
- un premier guide de coupe (1,100) comprenant des moyens de fixations (2) à un os (20) à découper en vue d'une ostéosynthèse ledit guide de coupe comprenant au moins deux fentes (3) définissant au moins deux plans de coupe;
- au moins un support (10,110) de fragment osseux (30), ledit support (10,110) de fragment osseux comprenant des moyens de fixation (16) au fragment osseux ;
- des moyens de positionnement univoque (14, 15) du au moins un support (10,110) de fragment osseux (30) par rapport à un os à traiter, ledit guide de coupe (1,100) comprenant des moyens de positionnement univoque (4,5,104) coopérant avec des moyens de positionnement univoque (14, 15, 114) correspondants sur le au moins un support (10,110) de fragment osseux (30).

2. Kit d'ostéotomie et de positionnement de fragments osseux selon la revendication 1 dans lequel ledit premier guide de coupe comprend des moyens de fixation amovible du au moins un support (10, 110) de fragment osseux (30).

3. Kit d'ostéotomie et de positionnement de fragments osseux selon l'une des revendications précédentes dans lequel les moyens de positionnement univoque du au moins un support (10, 110) de fragment osseux (30) comprennent un guide de reconstruction (70, 120) comprenant :
- des moyens de positionnement univoque (74,75) coopérant avec les moyens de positionnement univoque (14,15) correspondant sur le au moins un support (10, 110) de fragment osseux (30) et des moyens de fixation amovible du au moins un support (10, 110) de fragment osseux (30) ;
- et des moyens de fixation dans une position prédéfinie à un os à traiter(50).

4. Kit d'ostéotomie et de positionnement de fragments osseux selon la revendication 3 dans lequel les moyens de fixation dans une position prédéfinie à un os à traiter (50) comprennent des éléments d'ancrage (60) destinés à être fixés à une position prédéfinie sur un os à traiter (50) et permettant une fixation amovible dans une position prédéterminée du guide d'ostéosynthèse (70,120) aux éléments d'ancrage (60).

5. Kit d'ostéotomie et de positionnement de fragments osseux selon la revendication 4 comprenant un second guide de coupe (40) pouvant être fixé de façon amovible dans une position prédéterminée sur les éléments d'ancrage (60).

6. Kit d'ostéotomie et de positionnement de fragments osseux selon l'une des revendications précédentes dans lequel les moyens de fixation amovible sont sélectionnés parmi le groupe consistant en moyens d'encliquetage, moyens d'enclipsage, moyens de fixation par pression et système tenon/mortaise.

7. Kit d'ostéotomie et de positionnement de fragments osseux selon l'une des revendications précédentes comprenant une pluralité de supports (10, 110) de fragments osseux (30), les moyens de positionnement univoque (14,15) de chacun de ces supports (10, 110) étant différents les uns des autres.

8. Méthode de fabrication d'un kit d'ostéotomie et de positionnement de fragments osseux selon l'une des revendications précédentes comprenant les étapes de :
a) obtenir une représentation 3D d'un os à reconstruire (50) ;
b) obtenir une représentation 3D d'un os pour ostéosynthèse (20) ;
c) définir un plan chirurgical comprenant des plans de coupe de l'os à reconstruire (50) et de l'os pour ostéosynthèse (20), lesdits plans de coupe définissant des fragments osseux, et le plan chirurgical comprenant le positionnement relatifs de l'os à reconstruire d'une part et des fragments (30) d'osseux d'autre part;
d) déterminer la forme 3D d'un premier guide de coupe (1) comprenant une surface de positionnement sur l'os pour ostéosynthèse (20) et des fentes correspondantes aux plans de coupe définis à l'étape (c);
e) déterminer la forme 3D d'au moins un support (10, 110) de fragment osseux (30), positionné de façon univoque sur le premier guide de coupe (1), et ayant donc une position prédéterminée par rapport aux fragments osseux (30), la forme 3D du support de fragment comprenant des moyens de positionnement univoque du au moins un fragment osseux par rapport à l'os à reconstruire;
f) produire les formes 3D du premier guide de coupe et des supports de fragments osseux.

9. Méthode de fabrication selon la revendication 8 dans laquelle la production des formes 3D est effectuée par un procédé d'impression 3D.
